# EUROPEAN PATENT SPECIFICATION

(11) **EP 3 641 586 B1**
(45) Date of publication and mention of the grant of the patent: **30.04.2025**
(21) Application number: 18820142.0
(22) Date of filing: 22.06.2018
(51) Int. Cl.: A45D 6/10, A45D 6/12, A45D 6/14, A45D 7/02, A45D 7/04, A45D 7/06, A45D 2/00, A45D 20/12, A45D 20/50, A45D 34/04, A45D 1/04, A45D 24/22, A61N 5/06

(54) **A LIGHT-BASED SYSTEM AND METHOD FOR SHAPING AND TREATING HAIR**
LICHTBASIERTES SYSTEM UND VERFAHREN ZUM FORMEN UND BEHANDELN VON HAAREN
SYSTÈME REPOSANT SUR LA LUMIÈRE ET PROCÉDÉ DE MISE EN FORME ET DE TRAITEMENT DES CHEVEUX

(30) Priority: 22.06.2017 US 201762523734 P; 23.06.2017 US 201762523994 P; 13.09.2017 US 201762558016 P
(43) Date of publication of application: 29.04.2020
(73) Proprietor: The General Hospital Corporation, Boston, MA 02114 (US)
(72) Inventor: AUSTEN, William G., Boston, MA 02114 (US); REDMOND, Robert, Boston, MA 02114 (US); MCCORMACK, Michael, Boston, MA 02114 (US)
(74) Representative: Maiwald GmbH
(86) International application number: PCT/US2018/039134
(87) International publication number: WO 2018/237346

(56) References cited:
- WO-A2-2012/012050
- DE-A1- 102012 210 272
- FR-A1- 2 939 285
- US-A1- 2008 066 773
- US-A1- 2009 252 697
- US-A1- 2014 102 469
- US-A1- 2015 174 432
- US-A1- 2015 297 496
- US-B1- 7 699 058

## Description

### CROSS-REFERENCE TO RELATED APPLICATIONS

This application is based on, claims the benefit of U.S. Provisional Patent Application Serial No. 62/523,734, filed on June 22, 2017; U.S. Provisional Patent Application Serial No. 62/523,994, filed on June 23, 2017; and U.S. Provisional Patent Application Serial No. 62/558,016, filed on September 13, 2017.

### BACKGROUND

In the field of beauty and cosmetics, consumers are constantly seeking more efficient products and more effective tools for styling their hair. Such products might allow the consumer to more easily attain a style, such as straight or curly hair, while also offering improvements in volume, elasticity, and/or hold.

Currently, many hair shaping tools and products exist on the market that can generally be separated between temporary and permanent shaping methods. Each of these methods relies on disrupting and reorganizing the intermolecular interactions that are present within hair. For example, hair typically comprises fibrous proteins, such as α-keratin fibers, configured in a coiled coil secondary structure. Intermolecular interactions (i.e. hydrogen bonding, coulombic interactions, etc.) between amino acid side chains and covalent disulfide bonds between fibers dictate the secondary structure of the proteins present in hair. A change in the macromolecular structure of hair (i.e. from curly to straight) can be achieved by disrupting these stabilizing forces.

Temporary shaping methods typically disrupt the stabilizing forces present within hair through the application of heat. Heat is often applied through an apparatus, such as a blow dryer or a flat iron, at a temperature sufficient to disrupt the intermolecular interactions between the fibrous proteins. Sufficient temperatures typically range between 200 to 500 degrees Fahrenheit. During the temporary shaping method, heat is applied to the hair while simultaneously stretching or curling the hair into a desired shape. Heat is then removed, and the hair is allowed to cool in the desired shape to allow the intermolecular interactions to reform between the fibrous proteins, thereby providing hold to the new shape. Temporary shaping methods can also include applying hair-fixture polymers to the hair, such as waxes, gels, or pomades. A drawback of the temporary shaping method is that exposure to external forces, such as wind, humidity, or contact with water cause the hair to revert back to its natural shape. Furthermore, heating at elevated temperatures may cause damage to the hair.

So-called "permanent" shaping methods typically disrupt the stabilizing forces present within hair through the use of chemical reactions. In particular, permanent shaping methods use strong reducing agents to break the strong disulfide bonds present, for example, in the α-keratin fibers, followed by a neutralization step (i.e. application of hydrogen peroxide) to reform the disulfide bonds in the desired shape. Treatments typically include applying a high pH solution containing an alkaline hydroxide, such as NaOH, to the hair to induce disulfide bond reduction. Other strong reducing agents include formaldehyde, glycolic acid, and thioglycolic acid. A drawback of the permanent shaping method is that the procedure is time-consuming and can damage the hair due to the exposure to caustic chemicals. Additionally, should the consumer wish to revert from, for example, curly hair to straight hair, the procedure must be repeated or the hair allowed to grow the replace the hair that was treated. Over-exposure to the reducing agents further deteriorates the health of the hair.

French patent application FR 2 939 285 A1 discloses a method involving modifying a cross-section of a portion of a human or animal keratin fiber (F) by crushing and/or stretching of the fiber, and exposing the portion of the keratin fiber to a light pulse to provide a residual deformation to the portion of the keratin fiber, where the portion of the keratin fiber conserves the cross-section in which the fiber is drawn. The portion cross-section modifying process is carried out by exposition of the external pressure different from the atmospheric pressure.

United States patent US 7,699,058 B1 discloses a method for hair styling that includes generating pulses of light and directing the pulses of light towards hair fibers of an individual. The pulses have at least one pulse duration and a total energy all predetermined to effectively vary a characteristic of the hair fibers owing to absorption of light of the pulses by the hair fibers.

Therefore, it would be desirable to provide new systems and methods for shaping hair or providing aesthetic treatments to other parts of the body to provide new, yet flexible control to the individual. Additionally, it would be desirable to provide new systems and methods for shaping and treating hair without heat or potentially harmful chemicals.

### SUMMARY

In accordance with one aspect of the present invention, a method according to claim 1 is provided for performing a cosmetic treatment.

In accordance with another aspect of the present invention, a light-based system according to claim 3 is provided for treating and shaping a keratin fiber.

The foregoing and other aspects and advantages of the invention will appear from the following description. In the description, reference is made to the accompanying drawings, which form a part hereof, and in which there is shown by way of illustration a preferred embodiment of the invention. Such embodiment does not necessarily represent the full scope of the invention, however, and reference is made therefore to the claims for interpreting the scope of the invention.

### BRIEF DESCRIPTION OF THE DRAWINGS

The present invention will hereafter be described with reference to the accompanying drawings, wherein like reference numerals denote like elements.
Fig. 1 is a flowchart setting forth the steps of a method for performing a cosmetic treatment according to one aspect of the present disclosure.
Fig. 2 is a block diagram of a light-based system that is configured to implement a method for performing a cosmetic treatment in accordance with one aspect of the present disclosure.
Fig. 3 is an example cosmetic device that is configured to implement a method for performing a cosmetic treatment in accordance with one aspect of the present disclosure.
Fig. 4 is an example cosmetic device that is configured to implement a method for performing a cosmetic treatment in accordance with one aspect of the present disclosure.
Fig. 5 is an example cosmetic device that is configured to implement a method for performing a cosmetic treatment in accordance with one aspect of the present disclosure.
Fig. 6 is an example cosmetic device that is configured to implement a method for performing a cosmetic treatment in accordance with one aspect of the present disclosure.
Fig. 7 is an example cosmetic device that is configured to implement a method for performing a cosmetic treatment in accordance with one aspect of the present disclosure.
Fig. 8 is an example cosmetic device that is configured to implement a method for performing a cosmetic treatment in accordance with one aspect of the present disclosure.
Fig. 9 is a non-limiting example of an image of a subject's hair prior to performing a cosmetic treatment in accordance with one aspect of the present disclosure.
Fig. 10 is a series of images illustrating the subject's hair of Fig. 9 after the cosmetic treatment, and non-limiting examples of retention of the cosmetic treatment after exposure to multiple washes.
Fig. 11 is a non-limiting example of an image of a subject's hair prior to performing a cosmetic treatment in accordance with one aspect of the present disclosure.
Fig. 12 is an image of the subject's hair of Fig. 11 after the cosmetic treatment.
Fig. 13 is a series of images illustrating the subject's hair and non-limiting examples of retention of the cosmetic treatment after exposure to multiple washes.

### DETAILED DESCRIPTION

Described herein are methods, systems, and kits for performing a cosmetic treatment, such as treating or shaping keratin fibers on a subject. Exemplary keratin fibers may be, for example, keratin fibers found in a subject's hair or nail. In general, the methods described herein include applying, or otherwise administering, a photosensitizer to a keratin fiber of a subject while the keratin fiber is in a first physical state, and subjecting the keratin fiber to electromagnetic radiation at an appropriate wavelength, energy, and duration to cause the keratin fiber to transition to a second physical state that is different than the first physical state. As used herein, the term "physical state" may refer to any measurable property of the keratin fiber within the subject. Exemplary physical states of the keratin fiber may include, but are not limited to a mass, length, density, thickness, spatial orientation, volume, tensile strength, and/or color of the subject's hair or nail.

As used herein, the term "shaping" may relate to performing a cosmetic treatment on a keratin fiber to, for example, change the physical appearance of the subject's hair or nail. Shaping the keratin fibers may include applying, or otherwise administering, a photosensitizer to a keratin fiber in a region of interest of the subject, and irradiating the keratin fiber with electromagnetic radiation at an appropriate wavelength, energy, and duration to effectuate photocrosslinking on the keratin fiber, thereby transforming the keratin fiber from a first physical state to a second physical state. In some aspects of the disclosure, a cosmetic device (e.g., brush, hair drier, curling iron, straightening iron, nail applicator) is used to assist in physically transforming the keratin fiber from the first physical state to the second physical state. In one aspect of the disclosure, altering the keratin fiber from the first physical state to the second physical state includes altering the spatial orientation of the keratin fiber and photocrosslinking the keratin fiber while it is in the second physical state such that it retains the spatial orientation of the second physical state for a period of time. Altering the spatial orientation of the keratin fiber may include changing a subject's hair from straight to curly, curly to straight, and spatial orientations therebetween.

As used herein, the term "retain" generally refers to maintaining the second physical state (e.g., curly or straight) of the keratin fibers for a period of time. Such period of time may be one appropriate for hair applications, such as at least a few hours or extending indefinitely, until the keratin fibers are arranged in a different physical state. For example, the period of time may be measured based on a number of washes, where the second physical state of the keratin fibers may be substantially retained after being submerged and agitated (e.g., scrubbed) in water or soapy water. In some aspects of the disclosure, at least a portion of the keratin fibers can retain the second physical state over a period of time where at least 10% of the second physical state is retained after a number of washes, or at least 20 % of the second physical state, or at least 30% of the second physical state, or at least 40% of the second physical state, or at least 50% of the second physical state, or at least 60% of the second physical state, or at least 70% of the second physical state, or at least 80% of the second physical state, or at least 90% of the second physical state, or at least 95% of the second physical state, or more is retained after exposure to a number of washes, such as 1 wash, or 2 washes, or 3 washes, or 4 washes, or 5 washes, or 6 washes, or 7 washes, or 8 washes, or 9 washes, or 10 washes, or more.

As one non-limiting, illustrative example, the relative retention of the spatial orientation of the second physical state (e.g., curly or straight hair) after a number of washes may be monitored using the following procedure. First, a strand of hair in the second physical state may be extracted from the region of interest. One end of the strand of hair may then be fixed to a location, and the strand of hair may then be stretched until it is substantially linear. The length displacement between the resting state and stretched state is recorded. The strand of hair is then washed in water or soapy water, dried, and the measurement is repeated. The relative retention of the second physical state may then be recorded by comparing the displacement measurement before washing to the displacement measurements after washing.

In some aspects of the disclosure, altering the keratin fiber from the first physical state to the second physical state may include increasing the mass, length, density, thickness, volume, tensile strength, and/or color of the subject's hair or nail. In some forms, an exogenous fiber (e.g., fibrous protein or synthetic fiber) may be added to the region of interest prior to altering the keratin fiber from the first physical state to the second physical state. For example, prior to applying electromagnetic radiation to the target region, the exogenous fiber can be added to the photosensitizer solution and the keratin fiber in the target region. Alternatively or additionally, the exogenous fiber may be applied as a solid, for example, a powder or in the form of hair extensions. Electromagnetic radiation can then be applied to the target region to effectuate photocrosslinking between the fibrous protein and the keratin fiber in the region of interest such that the keratin fiber transforms from a first physical state to a second physical state. The second physical state of the keratin fiber may have a mass, mean diameter, volume, density, tensile strength, or length that is greater than the first physical state of the keratin fiber. In other aspects, the second physical state of the keratin fiber may comprise a color that is different than the color of the first physical state.

A suitable exogenous fibrous protein may comprise keratin, collagen, synthetic fibers, artificial hair, hair, derivatives and mixtures thereof. In one non-limiting example the synthetic fibers comprise vinyl chloride, modacrylic, vinylidene chloride, polyester, nylon, derivatives and mixtures thereof. The exogenous fibrous protein or synthetic fiber may be in the form of powders or hair extensions. Exemplary uses of the cosmetic treatment may include photocrosslinking the fibrous protein to a subject's nail to increase the density of the nail. Similarly, the fibrous protein may be photocrosslinked to the subject's hair to thicken thinning regions or to increase the length (i.e. attach a hair extension). Previous hair extension methods require keratin-based glues, heat, or ultrasonic waves to implement the extension. These previous methods further require long hours (i.e. 3-4 hours) and extensive maintenance. Unlike previous methods, the present disclosure provides a safer and faster application time that be performed without the addition of heat or potentially harmful chemicals. For example, the fibrous protein may be photocrosslinked to the keratin fiber in the target region in an application time of about 30 minutes, or 15 minutes, or 10 minutes, or 5 minutes, or 2 minutes, or less than 1 minute. In some aspects of the disclosure, the exogenous fiber may be suspended, or otherwise dissolved, in a biocompatible buffer or solution at a concentration of 0.01% (w/w) exogenous fiber to solution, or 0.05% (w/w), or 0.1% (w/w), or 0.5% (w/w), or 1% (w/w), or 2% (w/w), or 3% (w/w), or 4% (w/w), or 5% (w/w), or 6% (w/w), or 7% (w/w), or 8% (w/w), or 9% (w/w), or 10% (w/w), or more.

The fibrous protein may comprise a color such as blonde, brunette, brown, black, red, gray, platinum, or the like. The fibrous protein may be photocrosslinked to the keratin fiber in the target region to alter the color of the target region.

In some aspects of the disclosure, the retention of the second physical state relative to the first physical state can be measured explanting a keratin fiber from the region of interest while the keratin fibers are in the first physical state and performing a measurement, explanting a keratin fiber from the region of interest while the keratin fibers are in the second physical state and performing a measurement, and monitoring the changes in the measurements between the first physical state and the second physical state. In some aspects of the disclosure, at least a portion of the keratin fibers can retain the second physical state over a period of time where at least 10% of the second physical state is retained relative to the first physical state, or at least 20 % of the second physical state, or at least 30% of the second physical state, or at least 40% of the second physical state, or at least 50% of the second physical state, or at least 60% of the second physical state, or at least 70% of the second physical state, or at least 80% of the second physical state, or at least 90% of the second physical state, or at least 95% of the second physical state, or more is retained relative to the first physical state for a period of time, for example, for 1 day, or 1 week, or 2 weeks, or 3 weeks, or 4 weeks, or 1 month, or 2 months, or 3 months, or 4 months, or 5 months, or 6 months, or 7 months, or 8 months, or 9 months, or 10 months, or 11 months, or 1 year, or more. Suitable measurements to monitor the changes in the physical state from the first physical state to the second physical state include weight measurements, volume displacement measurements, colorimetry measurements, length measurements, and/or tensile strength measurements of the keratin fiber.

Alternatively or additionally, the physical state of the keratin fiber may also relate to an identifiable condition of a disease, or a concentration of a chemical species indicative of the disease. For example, nail diseases (e.g., onychosis) may be identified based on signs of infection or inflammation. Exemplary physical states may also include, but are not limited to, discoloration of the nail (e.g., yellowing, browning, and redness), shape and texture of the nail (degree of nail clubbing, koilonychias, pitting due to psoriasis, beau's lines), and pliability of nail (brittleness measured by iron concentration, splitting and fraying associated with folic acid, protein, and vitamin C deficiencies).

As used herein, the term "treating" may relate performing a cosmetic treatment to induce therapeutic changes. In some aspects of the disclosure, a therapeutically effective amount or pharmaceutically appropriate dosage of a photosensitizer is applied to a keratin fiber in the region of interest, and the keratin fiber is irradiated with electromagnetic radiation at an appropriate wavelength, duration, and intensity to elicit a biological or medical response of a subject, tissue, or cell that is being sought by the researcher, veterinarian, medical doctor, or other clinician. In some aspects of the disclosure, the biological or medical response is elicited by photoactivating the photosensitizer. In some aspects of the disclosure, the altering the keratin fiber from the first physical state to the second physical state may include reducing the degree or intensity of the identifiable condition of a disease or the concentration of a chemical species indicative of the disease, for example reducing the amount of fungus within the subject's nail, or reducing the amount of lice in the subject's hair. The term "subject" as used herein may refer to both human subjects and other animal subjects including domestic large and small animals such as dogs, cats, rabbits, horses, cows, pigs, and the like.

As used herein, "photoactivation" is used to describe the process by which energy in the form of electromagnetic radiation is absorbed by a compound, such as a photosensitizer. The electromagnetic radiation can include energy, e.g., light, having a wavelength in the visible range or portion of the electromagnetic spectrum, or the ultra violet and infrared regions of the spectrum. The chemical energy can be in the form of reactive species, such as a singlet oxygen, superoxide anion, hydroxyl radical, the excited state of the photosensitizer, photosensitizer free radical, or substrate free radical species. The photoactivation process described herein may involve insubstantial transfer of the absorbed energy into heat energy. Preferably, photoactivation occurs with a rise in temperature of less than 15 degrees Celsius, or a rise of less than 10 degrees Celsius, or a rise of less than 3 degrees Celsius, or a rise of less than 2 degrees Celsius, or a rise in temperature of less than 1 degree Celsius as measured, e.g., by an imaging thermal camera that looks at the target region during irradiation. The camera can be focused in the area of original dye deposit, e.g., target region comprising a keratin fiber, or on an area immediately adjacent the target region, to which dye will diffuse.

According to the invention, the term "photosensitizer" includes a chemical moiety that absorbs electromagnetic radiation, by a process such as photoactivation, to effectuate the formation of a covalent bond between two different parts of an individual keratin fiber.

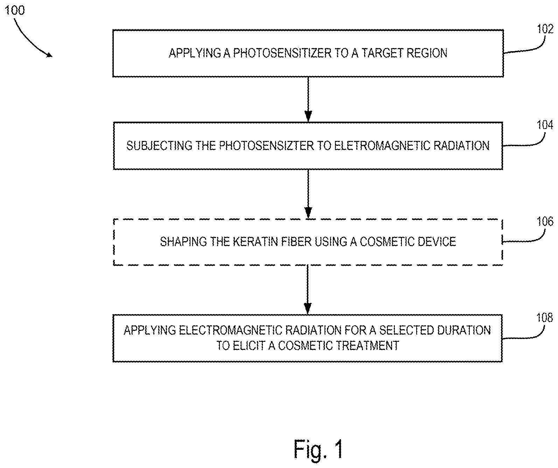

Referring particularly now to Fig. 1, an example method of performing a cosmetic treatment 100 is shown. The method includes applying, or otherwise administering, a photosensitizer 102 to a keratin fiber of a subject. The photosensitizer may be applied 102 manually, for example, using a spray bottle. Alternatively or additionally, the photosensitizer may also be applied 102 using a fluid dispensing system, for example, using a pump, nozzle, fluid supply tank, and a control unit to regulate the flow and dispensing rate. The photosensitizer may be dissolved in a biocompatible buffer or solution, e.g., saline solution, and used at a concentration of from 0.1 mM to 10mM, or from 0.5 mM to 5 mM, or from 1 mM to 3 mM. Alternatively, the photosensitizer may be dissolved or suspended within a gel, such as a hydrogel or a silicone gel. The keratin fiber may be applied on an external surface or an internal surface of the subject. An amount of photosensitizer may be applied that is sufficient to cover at least a portion of the keratin fiber. For example, at least 10 µL of photosensitizer solution, or greater than 50 µL, 100 µL, 250 µL, 500 µL, or 1 ml, or more can be applied to the target region. In some aspects, the photosensitizer may be dissolved in the biocompatible buffer or solution at a concentration of 0.01% (w/w) photosensitizer to solution, or 0.05% (w/w), or 0.1% (w/w), or 0.5% (w/w), or 1% (w/w), or 2% (w/w), or 3% (w/w), or 4% (w/w), or 5% (w/w), or 6% (w/w), or 7% (w/w), or 8% (w/w), or 9% (w/w), or 10% (w/w), or more.

Next, electromagnetic radiation is applied 104 to the keratin fiber and the photosensitizer. The electromagnetic radiation may be applied 104 at an appropriate wavelength, energy, and duration, to cause the photosensitizer to effectuate photocrosslinking on the keratin fiber, thereby transforming the keratin fiber from a first physical state to a second physical state. In some aspects of the disclosure, the electromagnetic radiation may be applied 104 at a therapeutically effective amount, or a pharmaceutically appropriate dosage, to elicit a biological or medical response to treat a disease or condition in the region of interest, thereby transforming the keratin fiber from a first physical state to a second physical state. In some aspects, a cosmetic device may be used to assist in shaping the keratin fiber 206 from the first physical state to the second physical state, such as by applying a force or/and or changes in temperature. This may involve, for example, shaping the hair using a brush or a curling iron prior to or during the application of electromagnetic radiation to the keratin fiber. The electromagnetic radiation may be applied for a selected duration 108 to elicit a response, such as to effectuate photocrosslinking on the keratin fiber or to induce photoactivation to treat a disease or condition. For example, the duration of irradiation can be from about 1 second to 30 minutes. In other aspects, the duration of irradiation ranges from about 1 second to 30 seconds, or 30 seconds to 2 minutes, or 2 minutes to 5 minutes, or greater than 5 minutes. The wavelength of light can be chosen so that it corresponds to the absorption of the photosensitizer, and reaches the area of the keratin fiber that has been contacted with the photosensitizer, e.g., penetrates into the region where the photosensitizer presents. The light source may be configured to apply electromagnetic radiation at a radiant energy that is less than 2000 J/cm², or between 50 and 200 J/cm². In some aspects, the light source may be configured to apply electromagnetic radiation at a radiant energy between 60 to 120 J/cm², or 80 to 100 J/cm². The electromagnetic radiation necessary to achieve photoactivation of the photosensitizer agent can have a wavelength from about 350 nm to about 800 nm, or from about 400 to 700 nm and can be within the visible, infrared or near ultraviolet spectra. The energy can be delivered at an irradiance of about between 0.1 and 5 W/cm², or between about 0.5 and 2 W/cm².

In some aspects, the second physical state may be tunable to range from a temporary transformation, a semi-permanent transformation, or a permanent transformation. In the context of a hair strand, determining whether a transformation is temporary or permanent may be measured as the hair's resistance to change after being exposed to moisture, such as a number of washes using water. A temporary transformation may be defined as a keratin fiber that reverts from the second physical state to the first physical state after exposure to 1 wash. A permanent transformation may be defined as a keratin fiber that that does not revert from the second physical state to the first physical state after exposure to 10 washes. A semi-permanent transformation falls within these bounds. The state of the transformation may be tuned, for example, by altering the wavelength, energy, or duration of electromagnetic radiation applied to the target region. Alternatively, the state of the transformation may be tuned by changing the concentration of the photosensitizer in the target region. In some aspects, the light-based system 100 may effectuate photocrosslinking in a keratin fiber such that the second physical state of the keratin fiber is resistant to at least 1 wash, at least 2 washes, at least 3 washes, at least 4 washes, at least 5 washes, at least 6 washes, at least 7 washes, at least 8 washes, at least 9 washes, or at least 10 washes.

In one non-limiting example method, a gel comprising the photosensitizer can be applied or administered to a subject's eyelid. A cosmetic device, such as a mascara brush, may then be used to brush a subject's eyelashes into the gel to place the keratin fibers positioned within the subject's eyelashes in contact with the photosensitizer. Alternatively, the gel may be applied or administered without the photosensitizer, and the photosensitizer may be applied manually to the subject's eyelashes by any of the methods described above. The subject's eyelashes may be configured to stick to the gel, or the eyelashes may be pulled away for further treatment prior to contacting the keratin fibers with the photosensitizer. A light source may then apply electromagnetic radiation to the subject's eyelashes to effectuate photocrosslinking on the keratin fibers, thereby transforming the keratin fiber from a first physical state to a second physical state. A protective cover can be used during treatment to block the electromagnetic radiation from entering the eye. In another non-limiting example, the cosmetic device may be used to brush the gel comprising the photosensitizer into the subject's eyelashes. The subject's eyelashes may then be stuck to the subject's eyelid or pulled away prior to treatment. Suitable gels include hydrogels, silicone gels, or the like. In some aspects, the gel may function as an optical wave guide to assist in delivering the electromagnetic radiation to the keratin fibers. For example, the silicone gel may comprise a reflective base that reflects the electromagnetic radiation and guides the electromagnetic radiation to the keratin fibers.

Suitable photosensitizers include, but are not limited to, a fluorescein, such as Rose Bengal (RB) (e.g., 4,5,6,7-tetrachloro-2',4',5',7'-tetraiodofluorescein or derivatives thereof); riboflavin (e.g., 7, 8-Dimethyl-10-[(2S,3S,4R)-2 ,3 ,4, 5-tetrahydroxypentyl]benzo[g]pteridine-2,4-dione or derivatives thereof); riboflavin-S-phosphate (R-5-P); methylene blue (MB); and N-hydroxypyridine-2-(1H)-thione (N-HTP), derivatives and mixtures thereof.

Other examples of photosensitive compounds that may be used in the fluid supply system 104 include various light-sensitive dyes and biological molecules such as, for example, Photofrin.RTM, synthetic diporphyrins and dichlorins, phthalocyanines with or without metal substituents, chloroaluminum phthalocyanine with or without varying substituents, O-substituted tetraphenyl porphyrins, 3,1-meso tetrakis (o-propionamido phenyl) porphyrin, verdins, purpurins, tin and zinc derivatives of octaethylpurpurin, etiopurpurin, hydroporphyrins, bacteriochlorins of the tetra(hydroxyphenyl) porphyrin series (e.g., protoporphyrin I through protoporphyrin IX, coproporphyrins, uroporphyrins, mesoporphyrins, hematoporphyrins and sapphyrins), chlorins, chlorin e6, mono-1-aspartyl derivative of chlorin e6, di-1-aspartyl derivative of chlorin e6, tin(IV) chlorin e6, meta-tetrahydroxphenylchlorin, benzoporphyrin derivatives, benzoporphyrin monoacid derivatives, tetracyanoethylene adducts of benzoporphyrin, dimethyl acetylenedicarboxylate adducts of benzoporphyrin, Diels-Adler adducts, monoacid ring "a" derivative of benzoporphyrin, sulfonated aluminum PC, sulfonated AlPc, disulfonated, tetrasulfonated derivative, sulfonated aluminum naphthalocyanines, naphthalocyanines with or without metal substituents and with or without varying substituents, chlorophylis, bacteriochlorophyll A, anthracenediones, anthrapyrazoles, aminoanthraquinone, phenoxazine dyes, thiazines, methylene blue, phenothiazine derivatives, chalcogenapyrylium dyes, cationic selena and tellurapyrylium derivatives, ring-substituted cationic PC, pheophorbide derivative, naturally occurring porphyrins, hematoporphyrin, ALA-induced protoporphyrin IX, endogenous metabolic precursors, 5-aminolevulinic acid, benzonaphthoporphyrazines, cationic imminium salts, tetracyclines, lutetium texaphyrin, texaphyrin, tin-etio-purpurin, porphycenes, benzophenothiazinium, xanthenes, rose bengal, eosin, erythrosin, cyanines, merocyanine 540, selenium substitued cyanines, flavins, riboflavin, proflavin, quinones, anthraquinones, benzoquinones, naphthaldiimides, naphthalimides, victoria blue, toluidine blue, dianthroquinones (e.g., hypericin), fullerenes, rhodamines and photosensitive derivatives thereof.

A suitable fibrous protein may comprise keratin, collagen, synthetic fibers, artificial hair, hair, and the like. In one non-limiting example the synthetic fibers comprise vinyl chloride, modacrylic, vinylidene chloride, polyester, nylon and mixtures thereof. Therapeutics may be coupled onto the fibrous protein prior to application to the target region. Therapeutics may include small molecule drugs, nucleic acid constructs (i.e. siRNA, aptamers, ribozymes, antisense oligonucleotides, and the like), and antibody constructs. In one non-limiting example, an antifungal medication may be coupled to the fibrous protein to provide a therapeutic effect. Example antifungal medications include clotrimazole, econazole, ketoconazole, miconazole, tioconazole, terbinafine, amorolfine, and the like.

### A Light-Based System:

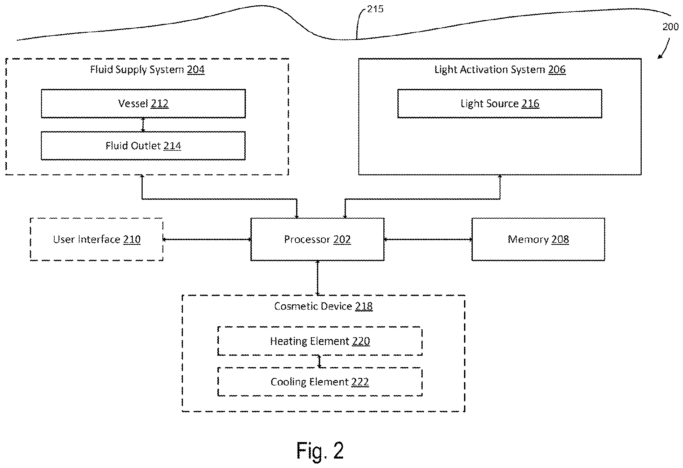

Referring particularly now to Fig. 2, an example of a light-based system 200 is illustrated that may perform the methods presented herein. In general, the light-based system 200 includes a processor 202 that is configured to be in electrical communication with a variety of components. The processor 202 may communicate with a pump or other components of a fluid supply system 204 and a light activation system 206. The optional fluid supply system 204 and the light activation system 206 or components thereof may be connected to the processor 202 by any suitable network connection, whether wired, wireless, or a combination of both. The processor 202 includes a commercially available programmable machine running on a commercially available operating system. That is, the processor 202 is configured with a memory 208 having stored programmable instructions therein. The processor 202 is capable of communicating with the fluid supply system 204 and the light activation system 206, processing data based on programmable instructions stored in the memory 208, and generating instructions. The processor 202 may be coupled to a user interface 210 that allows input parameters to be entered into the light-based system 200. The user interface 210 may be a switch or button or collection of switches or buttons. Also, the user interface 210 may include other interface components, such as displays or touch screens. To that end, the user interface 210 may also display the results.

The fluid supply system 204 includes a vessel 212 in fluid communication with a fluid outlet 214. The fluid supply system 204 is configured to dispense a fluid comprising a photosensitizer through the fluid outlet 214 towards a target region on a subject. The target region includes a keratin fiber 215 on the subject, such as a strand of hair or nail. In one aspect, the optional fluid supply system 204 functions in response to instructions from the processor 202 to dispense the fluid from the fluid outlet 212 to the target region on the subject. The processor 202 may regulate the flow rate of the fluid by communicating with a pump or valve within the fluid supply system 204. In some aspects, the fluid outlet 212 is coupled to a nozzle to dispense the fluid in a spray pattern, such as a conical mist, flat fan, or a steady stream. Alternatively, instead of the fluid supply system 104, a user can employ manual dispensing systems, such as a spray bottle, syringe, or pressurized vessel.

The light activation system 206 functions in response to instructions from the processor 202 to operate a light source 216 configured to apply electromagnetic radiation to the target region on the subject. Suitable light sources 216 include commercially available lasers, lamps, light emitting diodes, or other sources of electromagnetic radiation. Light radiation can be supplied in the form of a monochromatic laser beam, e.g., an argon laser beam or diode-pumped solid state laser beam. Light can also be supplied to a non-external surface tissue through an optical fiber device, e.g., the light can be delivered by optical fibers threaded through a small gauge hypodermic needle or an arthroscope. Light can also be transmitted by percutaneous instrumentation using optical fibers or cannulated waveguides.

In some aspects, the light-based system 100 includes a cosmetic device 118. The cosmetic device 118 is configured to assist in altering the keratin fiber from a first physical state to a second physical state such as by applying a force to the keratin fiber. The cosmetic device 118 may include a heating element 120 to assist in disrupting the non-covalent interactions within the keratin fiber, or a cooling element 122 to assist in reforming the non-covalent interactions. For example, if the keratin fiber is positioned within a strand of hair of the subject, the cosmetic device 118 may include a curling iron, a hair straightener, a blow dryer, a mascara brush, a hair brush, and the like. In the instance the keratin fiber is positioned within a nail of the subject the cosmetic device 118 may include a nail polish brush, sponge applicator, and the like.

In one aspect, the light-based system 100 may be useful in shaping a subject's hair to retain a particular physical appearance that is resistant to moisture and multiple washes. For example, the light-based system 100 can be used to alter a keratin fiber positioned within a subject's hair to change from a first physical state, e.g. straight hair, to a second physical state, e.g. curly hair, or vice versa. As described above, cosmetic devices 118 may be used to assist in shaping the hair prior to photocrosslinking.

Photocrosslinkng offers several benefits over conventional methods for shaping hair. First, the methods described herein may be performed using no heat or at a sufficiently low temperature to avoid heat induced damage to the hair. Second, unlike conventional hair shaping methods that rely on disrupting and reforming non-covalent interactions, the present disclosure forms strong covalent bonds between and within the keratin fibers that result in improved hair hold and resistance to external forces.

### Cosmetic Devices:

Various cosmetic devices 118 may be used with the light-based system 100 to assist in shaping or treating the keratin fiber. In some aspects of the disclosure, the cosmetic devices 118 may be configured to implement the methods described herein. The various devices are described below.

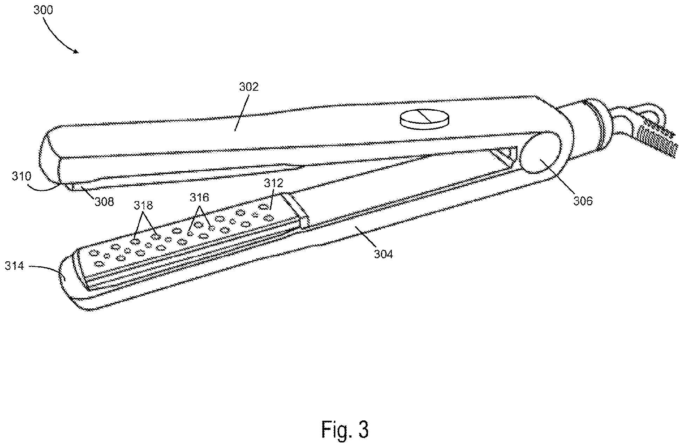

Referring to Fig. 3 an example of a cosmetic device 300 is illustrated that may be configured to implement the methods presented herein. In general, the cosmetic device 300 includes a first handle 302 and a second handle 304 extending from a hinge 306. The first handle 302 includes a first conductive element 308 attached on a first inner surface 310 of the first handle 302. The second handle 304 includes a second conductive element 312 attached on a second inner surface 314 of the second handle 304. At least one heating element may be placed in contact with the first conductive element 308 and the second conductive element 312. The cosmetic device 300 may include at least one fluid dispensing port 316 that is placed in fluid communication with the fluid supply system 204. In some aspects of the disclosure, the fluid supply system 204 may be configured within the cosmetic device 300. For example, the fluid supply system 204 may be configured within the first handle 302 or the second handle 304. Alternatively, the fluid supply system 204 may be configured external to the cosmetic device 300 and a conduit may place the dispensing port 316 in fluid communication with the fluid supply system 204. The dispensing port 316 may be configured to dispense the photosensitizer to a target region. In one aspect the target region is positioned between the first conductive element 308 and the second conductive element 312.

The cosmetic device 300 includes at least one light dispensing port 318 configured to the activation system 106. The light source 116 of the activation system 106 may be configured within the at least one light dispensing port 318 to apply electromagnetic radiation at the target region. The processor 202 may be placed in electrical communication with the at least one heating element to control the temperature of the first conductive element 308 and the second conductive element 312. In some aspects of the disclosure, the processor 202 is configured within the cosmetic device 300.

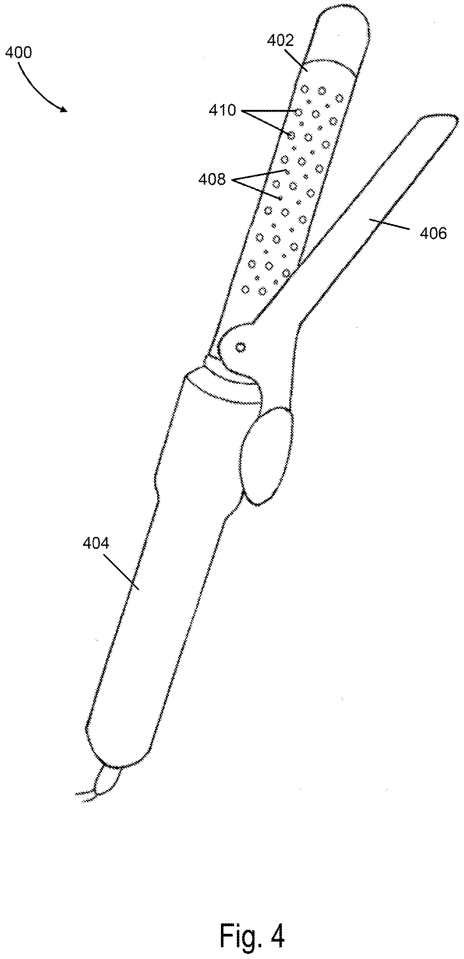

Referring to Fig. 4 an example of a cosmetic device 400 is illustrated that may be configured to implement the methods presented herein. The cosmetic device 400 includes a heat conducting body 402 having an exterior surface and a hollow center. The heat conducting body 402 may include at least one heating element disposed within the hollow center to heat the heat conducting body 402. A handle 404 is configured to the heat conducting body, wherein the handle 404 is composed of a material that does not substantially conduct heat. The cosmetic device 400 further includes a clamp member 406 pivotally connected to the heat conducting body 402. The clamp member 406 may be moved between an open position and a closed position, where the clamp member is configured to rest on the exterior surface of the heat conducting body 402 when the clamp member 406 is in the closed position. The cosmetic device includes at least one fluid dispensing port 408 that is placed in fluid communication with the fluid supply system 204. For example, the fluid supply system 204 may be configured within the handle 404. Alternatively, the fluid supply system 204 may be configured external to the cosmetic device 400 and a conduit may place the dispensing port 416 in fluid communication with the fluid outlet 214 of the fluid supply system 204. The dispensing port 416 may be configured to dispense the photosensitizer to a target region. In one aspect the target region is positioned between the heat conducting body 402 and the clamp member 406.

The cosmetic device 400 includes at least one light dispensing port 410 configured to the activation system 206. The light source 216 of the activation system 206 may be configured within the at least one light dispensing port 410 to apply electromagnetic radiation at the target region. The processor 202 may be placed in electrical communication with the at least one heating element to control the temperature of the heat conducting body 402. In some aspects of the disclosure, the processor 202 is configured within the cosmetic device 400.

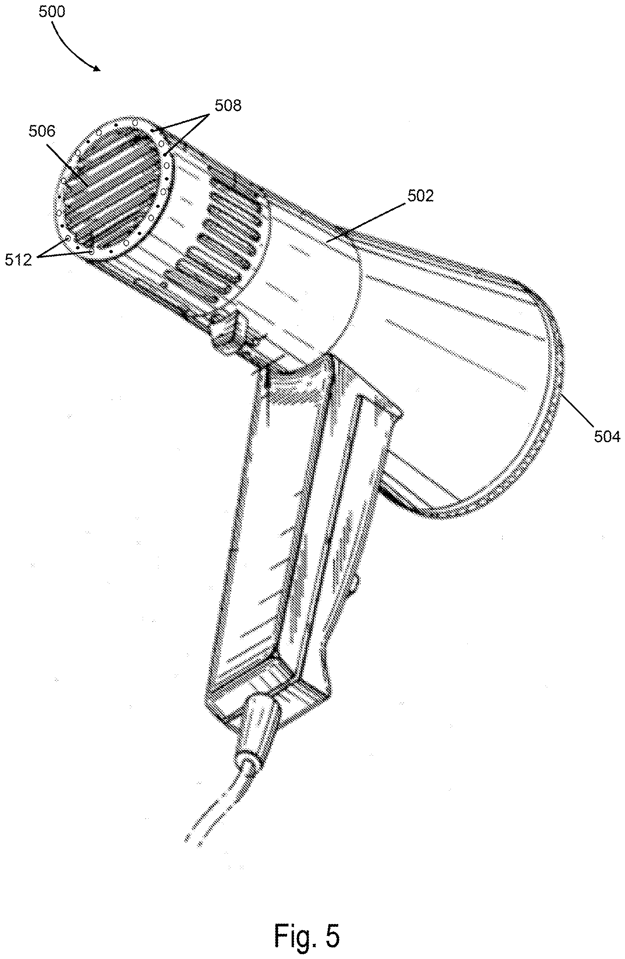

Referring to Fig. 5 an example of a cosmetic device 500 is illustrated that may be configured to implement the methods described herein. The cosmetic device 500 includes an elongated housing 502 extending from an inlet 504 to an outlet 506 to define an airflow axis. The cosmetic device 500 includes a fan positioned within the elongated housing 502 where the fan is configured to draw air into the elongated housing 502 through the inlet 504 and force air through the outlet 506 to a target region. In some aspects, the target region is defined along the airflow axis. The elongated housing 502 is configured with at least one heating element disposed within the housing. The cosmetic device 500 includes at least one fluid dispensing port 508 configured to the cosmetic device includes at least one fluid dispensing port 508 that is placed in fluid communication with the fluid supply system 204. For example, the fluid supply system 204 may be configured within a handle 510 configured to the elongated housing 502. Alternatively, the fluid supply system 204 may be configured external to the cosmetic device 500 and a conduit may place the dispensing port 508 in fluid communication with the fluid outlet 214 of the fluid supply system 204. The dispensing port 506 may be configured to dispense the photosensitizer to the target region.

The cosmetic device 500 includes at least one light dispensing port 512 configured to the activation system 206. The light source 216 of the activation system 206 may be configured to the at least one light dispensing port 512 to apply electromagnetic radiation at the target region. The processor 202 may be placed in electrical communication with the at least one heating element to control the temperature of air being delivered to the target region. In some aspects of the disclosure, the processor 202 is configured within the cosmetic device 500.

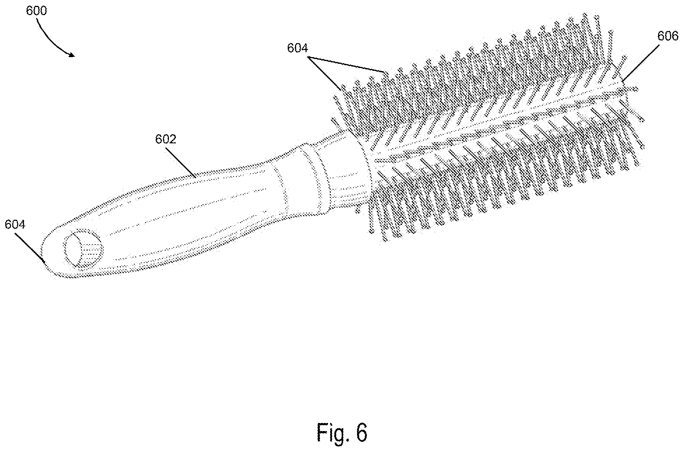

Referring to Fig. 6, an example of a cosmetic device 600 is illustrated that may be configured to implement the methods described herein. The cosmetic device 600 includes a handle 602 extending from a first end 604 to a second end 606. The handle 602 includes at least one filament 608 extending from the handle 602, for example, perpendicularly from an exterior surface of the handle 602. The at least one filament 608 is configured to the light source 216 of the activation system 206 to apply electromagnetic radiation at a target region. For example, the at least one filament 608 may be include an optical fiber or optical cable that may transmit electromagnetic radiation to the target region. In other aspects, the filament may comprise a light translucent material that transmits electromagnetic radiation to the target region. The activation system 106 may be configured within a hollow chamber positioned within the handle 602. In some aspects of the disclosure the at least one filament 608 may be coated with a photosensitizer, which may be in the form of a gel. The photosensitizer may then be transferred to the target region though the at least one filament 608. The cosmetic device 600 may include at least one fluid dispensing port configured to the cosmetic device 600 (e.g., in the at least one filament 608) includes at least one fluid dispensing port that is placed in fluid communication with the fluid supply system 204. For example, the fluid supply system 204 may be configured within the handle 602. Alternatively, the fluid supply system 204 may be configured external to the cosmetic device 600 and a conduit may place the dispensing port in fluid communication with the fluid outlet 214 of the fluid supply system 204. The dispensing port may be configured to dispense the photosensitizer to the target region.

In one aspect, the target region may be a distance from the at least one filament 604 wherein the distance is less than 1 cm, 2 cm, 3 cm, 4 cm, or 5 cm. In one aspect, the activation system 106 may be configured within a hollow center of the handle

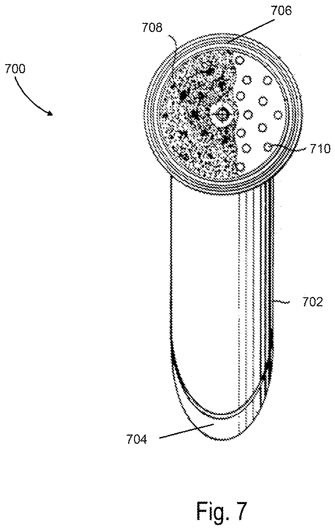

Referring to Fig. 7, an example of a cosmetic device 700 is illustrated. The cosmetic device 700 includes a handle 702 extending from a first end 704 to a second end 706. The second end 706 is configured to a porous media 708. The porous media being composed of a material that absorbs fluids, such as water or water-based solutions. For example, the porous media 708 may comprise cellulose wood fibers or foamed plastic polymers. The second end 706 is further configured with at least one light dispensing port 512 configured to the activation system 206. The light source 216 of the activation system 206 may be configured to the at least one light dispensing port 512 to apply electromagnetic radiation at the target region. The porous media 708 may be used to absorb a photosensitizer solution and apply it to a keratin fiber in a target region, such as a subject's nail.

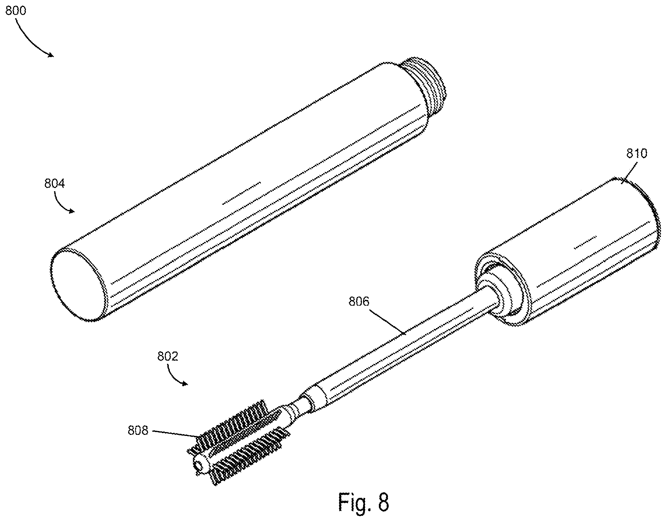

Referring to Fig. 8, an example of a cosmetic device 800 is illustrated. The cosmetic device 800 includes an applicator 802 and a container 804 that is configured to receive a photosensitizer 202. The photosensitizer 202 may be dissolved in a liquid solution, such as a saline solution. Alternatively, the photosensitizer 202 may be dissolved or suspended in a gel, such as a hydrogel or a silicone gel. The applicator 802 includes a stem 806 that extends between an applicator head 808 and a handle 810. The handle 810 is configured to be removeable coupled to a top face of the container 804, for example by a screw thread, and the stem 806 is configured to be disposed within the container 804 to place the applicator head 808 in contact with the photosensitizer 202. At least one filament 810 is configured to extend from an exterior surface of the applicator head 808. The at least one filament 810 is configured to the light source 216 of the activation system 206 to apply electromagnetic radiation at a target region. For example, the at least one filament 810 include an optical fiber or optical cable that transmits the electromagnetic radiation to the target region. In other aspects, the filament 810 may comprise a light translucent material that transmits electromagnetic radiation to the target region.

The systems and methods described herein are suitable for use in a variety of applications, including in vitro laboratory applications, ex vivo and in vivo keratin fiber treatments on living subjects.

### EXAMPLES

The following examples set forth, in detail, ways in which the system may be used or implemented, and will enable one of skill in the art to more readily understand the principles thereof. The following examples are presented by way of illustration and are not meant to be limiting in any way.

### Example 1:

In Example 1, a photosensitizer was used to increase the tensile strength of a subject's strand of hair. In the example, 0.1% Rose Bengal solution was applied to the strand of hair, and the hair was irradiated with green light. The load at break (Newtons) was measured for the single strain of hair with no wash and after multiple washes. The wash protocol included shampooing the hair, washing in water, conditioning the hair, and washing with water. The results are summarized below:

| | Control (N) | PXL (N) | % Diff | P Value |
|---|---|---|---|---|
| No Wash | 0.868 | 1.069 | -23% | 0.02 |
| One Wash | 0.787 | 0.969 | -23% | 0.05 |
| Two Wash | 0.753 | 0.974 | -29% | 0.01 |
| Combined | 0.803 | 1.004 | -25% | 0.0001 |
| Sample Size/Time Point | 15 | 15 | | |

### Example 2:

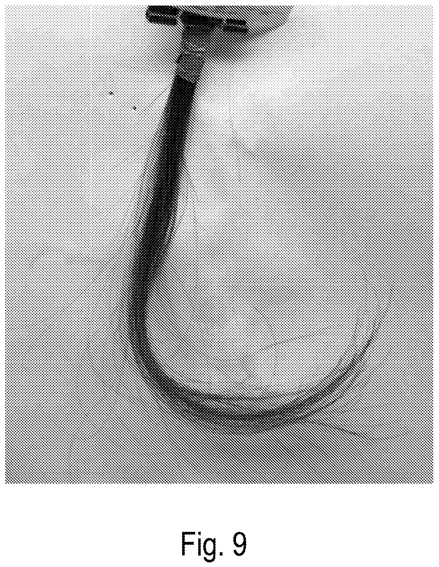

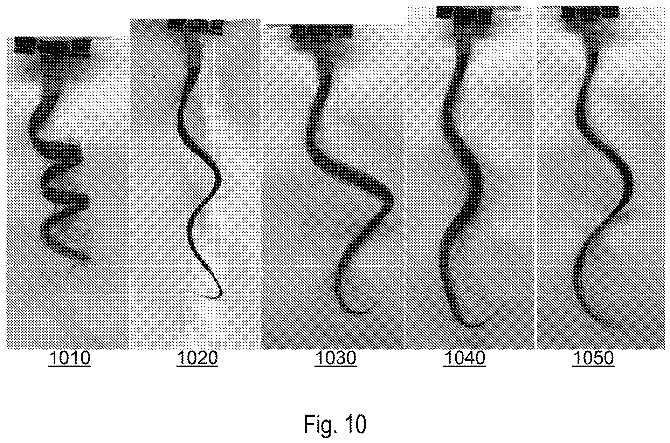

In Example 2, a solution comprising a photosensitizer and exogenous fiber was used to perform a cosmetic treatment on a subject's hair. FIG. 9 shows an image of the subject's hair prior to the cosmetic treatment. In the example, a phosphate-buffered saline (PBS) solution having a concentration of 4% (w/w) Keratin and 0.1% (w/w) Rose Bengal was applied to a subject's hair, and the hair was physically altered from its original shape into a curled state without the application of heat or other chemicals. The hair was then irradiated with electromagnetic radiation for a duration. FIG. 10 shows a series of images that illustrate the subject's hair after treatment 1010, after one wash 1020, after two washes 1030, after three washes 1040, and after four washes 1050. The washing protocol included shampooing the hair, and washing with water.

### Example 3:

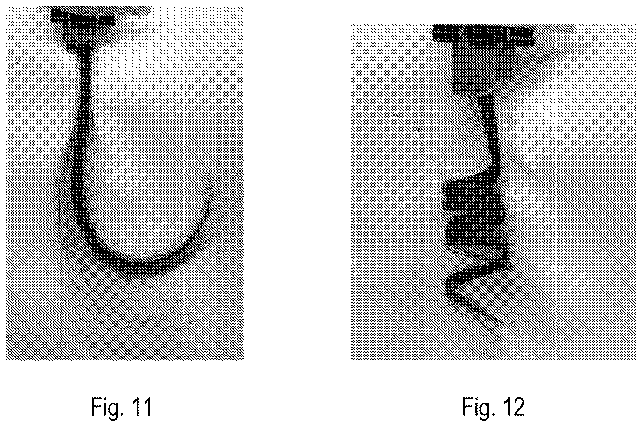

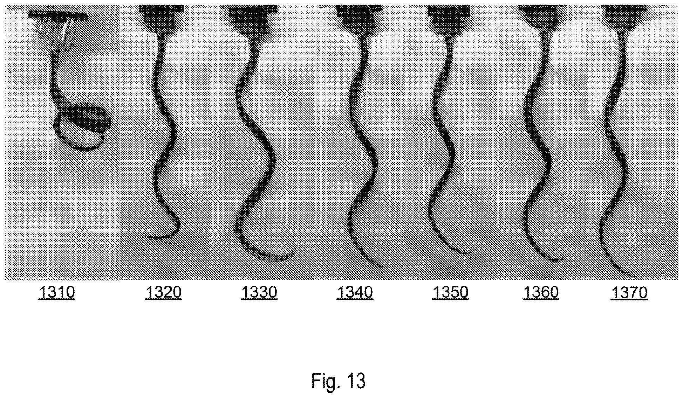

In Example 3, a solution comprising a photosensitizer and exogenous fiber was used to perform a cosmetic treatment on a subject's hair. FIG. 11 shows an image of the subject's hair prior to the cosmetic treatment. In the example, a phosphate-buffered saline (PBS) solution having a concentration of 8% (w/w) Keratin and 0.1% (w/w) Rose Bengal was applied to a subject's hair, and the hair was physically altered from its original shape into a curled state without the application of heat or other chemicals. The hair was then irradiated with electromagnetic radiation for a duration. FIG. 12 shows an image of the subject's hair after treatment, and FIG. 13 shows a series of images that illustrate the subject's hair after one wash 1310, after two washes 1320, after three washes 1330, after four washes 1340, after five washes 1350, after six washes 1360, and after eight washes 1370. The washing protocol included shampooing the hair, and washing with water.

The present invention has been described in terms of one or more preferred embodiments, and it should be appreciated that many equivalents, alternatives, variations, and modifications, aside from those expressly stated, are possible without departing the scope of the invention as defined in the appended claims.

## Claims

1. A method of performing a cosmetic treatment, the method comprising:
(a) applying a photosensitizer (102) to a keratin fiber of a subject while the keratin fiber is in a first physical state; and
(b) subjecting the keratin fiber and the photosensitizer to electromagnetic radiation (104) selected to effectuate formation of a covalent bond between two different parts of the individual keratin fiber to cause the keratin fiber to transition to a second physical state that is different than the first physical state.

2. The method of claim 1, wherein the keratin fiber is positioned within a strand of hair of the subject and wherein the method further comprises:
(a) applying a fibrous protein to the strand of hair on the subject;
(b) subjecting the fibrous protein, the photosensitizer (102), and the strand of hair to electromagnetic radiation (104) selected to effectuate photocrosslinking between the fibrous protein and the keratin fiber within the strand of hair; and
wherein the photocrosslinking transitions the keratin fiber from the first physical state to the second physical state.

3. A light-based system for treating and shaping a keratin fiber, the system (200) comprising:
a fluid supply system (204) comprising a vessel (212) in fluid communication with a fluid outlet (214);
an activation system (206) configured with one or more light source (216), the light source (216) configured to apply electromagnetic radiation at a target region that includes the keratin fiber; and
a processor (202) having a memory (208) and configured to be in electrical communication with the activation system (206), the processor (202) being configured to implement stored instructions from the memory (208) to cause the activation system (206) to apply the electromagnetic radiation to the target region to effectuate formation of a covalent bond between two different parts of the individual keratin fiber to cause a transition from a first physical state to a second physical state,
wherein
the vessel (212) is configured to contain a photosensitizer and the fluid outlet (214) is configured to dispense the photosensitizer at the target region.

4. The light-based system of claim 3, wherein the processor (202) is in electrical communication with a dispensing system to cause the dispensing system to dispense the photosensitizer to the target region to coat at least a portion of the keratin fiber with the photosensitizer.

5. The light-based system of claim 3, further comprising a cosmetic device (218) configured to apply a physical force to the keratin fiber in the target region to move the keratin fiber from the first physical state to the second physical state.

6. The light-based system of claim 5, wherein the cosmetic device (218) includes a heating element (220) or a cooling element (222).

7. The light-based system of claim 5, wherein the cosmetic device (218) comprises:
a pair of handles (302, 304) extending from a hinge (306), the pair of handles (302, 304) includes a first handle (302) and a second handle (304);
a first conductive element (308) attached on an inner surface (310) of the first handle (302);
a second conductive element (312) attached on an inner surface (314) of the second handle (304);
at least one heating element (220) in contact with the first conductive element (308) and the second conductive element (312);
at least one fluid dispensing port (316) configured to dispense the photosensitizer to the target region;
at least one light dispensing port (318) configured to apply electromagnetic radiation to the target region; and
wherein the processor (202) is in electrical communication with the at least one heating element (220) and is programmed to cause the first conductive element (308) and the second conductive element (312) to increase in temperature.

8. The light-based system of claim 5, wherein the cosmetic device (218) comprises:
a heat conducting body (402) having an exterior surface and a hollow center;
at least one heating element (220) disposed within the hollow center configured to heat the heat conducting body (402);
a handle (404) coupled to the heat conducting body (402);
a clamp (406) pivotally connected to the heat conducting body (402), the clamp (406) being movable between an open position and a closed position, wherein the clamp (406) is configured to rest on a surface of the heat conducting body (402) when the clamp (406) is in the closed position;
at least one fluid dispensing port (408) configured to deliver the photosensitizer to the target region;
at least one light dispensing port (410) configured to apply electromagnetic radiation at the target region; and
wherein the processor (202) is in electrical communication with the at least one heating element (220) to cause the at least one heating element (220) to raise in temperature.

9. The light-based system of claim 5, wherein the cosmetic device (218) comprises:
an elongated housing (502) extending from an inlet (504) to an outlet (506) to define an airflow axis;
a fan positioned within the elongated housing (502) to draw air into the elongated housing (502) through the inlet (504) and force air through the outlet (506) toward a target region;
at least one heating element (220) disposed within the housing (502);
at least one fluid dispensing port (506) configured to dispense the photosensitizer to the target region;
at least one light dispensing port (512) configured apply electromagnetic radiation at the target region;
the processor (202) in electrical communication with the at least one heating element (220) to cause the heating element (220) to increase a temperature of the airflow through the airflow axis.

10. The light-based system of claim 5, wherein the cosmetic device (218) comprises:
a handle (602) extending from a first end (604) to a second end (606);
at least one filament (608) extending from an exterior surface of the handle (602), wherein the light source (216) of the activation system (206) is configured to cause the at least one filament (608) to apply electromagnetic radiation to the target region.

11. The light-based system of claim 5, wherein the cosmetic device (218) comprises:
a handle (702) extending from a first end (704) to a second end (706);
a porous media (708) configured to the second end (706); and
at least one light dispensing port (512) configured to apply electromagnetic radiation at the target region.

12. The light-based system of claim 5, wherein the cosmetic device (218) comprises:
a container (804) configured to receive the photosensitizer;
an applicator (802) comprising a stem (806) that extends between an applicator head (808) and a handle (810), the handle (810) configured to be removeably coupled to a top face of the container (804) and the stem (806) configured to be disposed within the container (804); and
at least one filament (810) extending from an exterior surface of the applicator head (808), wherein the light source (216) of the activation system (206) is configured to cause the at least one filament (810) to apply electromagnetic radiation to the target region.

13. The light-based system of claim 12, wherein the photosensitizer is suspended or dissolved in a gel or is dissolved in a liquid solution.

14. The light-based system of claim 13, wherein the gel further comprises an exogenous fiber.

15. The light-based system of claim 13, wherein the liquid solution further comprises an exogenous fiber.

## Patentansprüche

1. Verfahren zum Durchführen einer kosmetischen Behandlung, wobei das Verfahren umfasst:
(a) Aufbringen eines Photosensibilisators (102) auf eine Keratinfaser eines Subjekts, während sich die Keratinfaser in einem ersten physikalischen Zustand befindet; und
(b) Aussetzen der Keratinfaser und des Photosensibilisators einer elektromagnetischen Strahlung (104), die so ausgewählt ist, dass sie die Bildung einer kovalenten Bindung zwischen zwei verschiedenen Teilen der einzelnen Keratinfaser bewirkt, um zu bewirken, dass die Keratinfaser in einen zweiten physikalischen Zustand übergeht, der sich von dem ersten physikalischen Zustand unterscheidet.

2. Verfahren nach Anspruch 1, wobei die Keratinfaser innerhalb einer Haarsträhne des Subjekts positioniert ist und wobei das Verfahren ferner umfasst:
(a) Auftragen eines faserigen Proteins auf die Haarsträhne der Person;
(b) Aussetzen des Faserproteins, des Photosensibilisators (102) und der Haarsträhne einer elektromagnetischen Strahlung (104), die ausgewählt ist, um eine Photovernetzung zwischen dem Faserprotein und der Keratinfaser innerhalb der Haarsträhne zu bewirken; und
wobei die Fotovernetzung die Keratinfaser von dem ersten physikalischen Zustand in den zweiten physikalischen Zustand überführt.

3. Lichtbasiertes System zur Behandlung und Formung einer Keratinfaser, wobei das System (200) aufweist:
ein Fluidversorgungssystem (204), das einen Behälter (212) in Fluidverbindung mit einem Fluidauslass (214) aufweist;
ein Aktivierungssystem (206), das mit einer oder mehreren Lichtquellen (216) ausgestattet ist, wobei die Lichtquelle (216) konfiguriert ist, dass elektromagnetische Strahlung auf einen Zielbereich anzuwenden, der die Keratinfaser enthält; und
einen Prozessor (202) mit einem Speicher (208), der konfiguriert ist, in elektrischer Verbindung mit dem Aktivierungssystem (206) zu stehen, wobei der Prozessor (202) konfiguriert, gespeicherte Anweisungen aus dem Speicher (208) zu implementieren, um das Aktivierungssystem (206) zu veranlassen, die elektromagnetische Strahlung auf den Zielbereich anzuwenden, um die Bildung einer kovalenten Bindung zwischen zwei verschiedenen Teilen der einzelnen Keratinfaser zu bewirken, um einen Übergang von einem ersten physikalischen Zustand zu einem zweiten physikalischen Zustand zu bewirken, wobei
das Gefäß (212) so konfiguriert ist, dass es einen Photosensibilisator zu enthält, und der Fluidauslass (214) konfiguriert ist, den Photosensibilisator an den Zielbereich abzugeben.

4. Lichtbasiertes System nach Anspruch 3, wobei der Prozessor (202) in elektrischer Verbindung mit einem Abgabesystem steht, um das Abgabesystem zu veranlassen, den Photosensibilisator in den Zielbereich abzugeben, um mindestens einen Teil der Keratinfaser mit dem Photosensibilisator zu beschichten.

5. Lichtbasiertes System nach Anspruch 3, ferner aufweisend eine kosmetische Vorrichtung (218), die konfiguriert ist, eine physikalische Kraft auf die Keratinfaser im Zielbereich auszuüben, um die Keratinfaser aus dem ersten physikalischen Zustand in den zweiten physikalischen Zustand zu bringen.

6. Lichtbasiertes System nach Anspruch 5, wobei die kosmetische Vorrichtung (218) ein Heizelement (220) oder ein Kühlelement (222) enthält.

7. Lichtbasiertes System nach Anspruch 5, wobei die kosmetische Vorrichtung (218) aufweist:
ein Paar von Griffen (302, 304), die sich von einem Scharnier (306) aus erstrecken, wobei das Paar von Griffen (302, 304) einen ersten Griff (302) und einen zweiten Griff (304) aufweist;
ein erstes leitendes Element (308), das an einer Innenfläche (310) des ersten Griffs (302) angebracht ist;
ein zweites leitendes Element (312), das an einer Innenfläche (314) des zweiten Griffs (304) angebracht ist;
mindestens ein Heizelement (220), das in Kontakt mit dem ersten leitenden Element (308) und dem zweiten leitenden Element (312) steht;
mindestens eine Fluidabgabeöffnung (316), die konfiguriert ist, den Photosensibilisator an den Zielbereich abzugeben;
mindestens eine Lichtabgabeöffnung (318), die konfiguriert ist, elektromagnetische Strahlung auf den Zielbereich aufzubringen; und
wobei der Prozessor (202) in elektrischer Verbindung mit dem mindestens einen Heizelement (220) steht und programmiert ist, das erste leitende Element (308) und das zweite leitende Element (312) zu veranlassen, ihre Temperatur zu erhöhen.

8. Lichtbasiertes System nach Anspruch 5, wobei die kosmetische Vorrichtung (218) aufweist:
einen wärmeleitenden Körper (402) mit einer Außenfläche und einem hohlen Kern;
mindestens ein Heizelement (220), das innerhalb des hohlen Zentrums angeordnet ist und konfiguriert ist, den wärmeleitenden Körper (402) zu erwärmen;
einen Griff (404), der mit dem wärmeleitenden Körper (402) verbunden ist;
eine Klemme (406), die schwenkbar mit dem wärmeleitenden Körper (402) verbunden ist, wobei die Klemme (406) zwischen einer offenen Position und einer geschlossenen Position beweglich ist, wobei die Klemme (406) so konfiguriert ist, dass sie auf einer Oberfläche des wärmeleitenden Körpers (402) aufliegt, wenn sich die Klemme (406) in der geschlossenen Position befindet;
mindestens eine Fluidabgabeöffnung (408), die konfiguriert ist, den Photosensibilisator an den Zielbereich abzugeben;
mindestens eine Lichtabgabeöffnung (410), die konfiguriert ist, elektromagnetische Strahlung auf den Zielbereich aufzubringen; und
wobei der Prozessor (202) in elektrischer Verbindung mit dem mindestens einen Heizelement (220) steht, um das mindestens eine Heizelement (220) zu veranlassen, seine Temperatur zu erhöhen.

9. Lichtbasiertes System nach Anspruch 5, wobei die kosmetische Vorrichtung (218) aufweist:
ein längliches Gehäuse (502), das sich von einem Einlass (504) zu einem Auslass (506) erstreckt, um eine Luftstromachse zu definieren;
ein Gebläse, das in dem länglichen Gehäuse (502) angeordnet ist, um Luft durch den Einlass (504) in das längliche Gehäuse (502) zu saugen und Luft durch den Auslass (506) in Richtung eines Zielbereichs zu drücken;
mindestens ein Heizelement (220), das in dem Gehäuse (502) angeordnet ist;
mindestens eine Fluidabgabeöffnung (506), die konfiguriert ist, den Photosensibilisator an den Zielbereich abzugeben;
mindestens eine Lichtabgabeöffnung (512), die konfiguriert ist, elektromagnetische Strahlung auf den Zielbereich aufzubringen;
der Prozessor (202) in elektrischer Verbindung mit dem mindestens einen Heizelement (220) steht, um das Heizelement (220) zu veranlassen, eine Temperatur des Luftstroms durch die Luftstromachse zu erhöhen.

10. Lichtbasiertes System nach Anspruch 5, wobei die kosmetische Vorrichtung (218) aufweist:
einen Griff (602), der sich von einem ersten Ende (604) zu einem zweiten Ende (606) erstreckt;
mindestens einen Glühfaden (608), der sich von einer Außenfläche des Griffs (602) erstreckt, wobei die Lichtquelle (216) des Aktivierungssystems (206) konfiguriert ist, den mindestens einen Glühfaden (608) zu veranlassen, elektromagnetische Strahlung auf den Zielbereich aufzubringen.

11. Lichtbasiertes System nach Anspruch 5, wobei die kosmetische Vorrichtung (218) aufweist:
einen Griff (702), der sich von einem ersten Ende (704) zu einem zweiten Ende (706) erstreckt;
ein poröses Medium (708), das am zweiten Ende (706) angeordnet ist; und
mindestens eine Lichtabgabeöffnung (512), die konfiguriert ist, elektromagnetische Strahlung auf den Zielbereich aufzubringen.

12. Lichtbasiertes System nach Anspruch 5, wobei die kosmetische Vorrichtung (218) aufweist:
einen Behälter (804), der konfiguriert ist, den Photosensibilisator aufzunehmen;
einen Applikator (802), der einen Stiel (806) aufweist, der sich zwischen einem Applikatorkopf (808) und einem Griff (810) erstreckt, wobei der Griff (810) so konfiguriert ist, dass er abnehmbar mit einer oberen Fläche des Behälters (804) gekoppelt ist, und der Stiel (806) so konfiguriert ist, dass er innerhalb des Behälters (804) angeordnet ist; und
mindestens einen Glühfaden (810), der sich von einer Außenfläche des Applikatorkopfes (808) erstreckt, wobei die Lichtquelle (216) des Aktivierungssystems (206) konfiguriert ist, den mindestens einen Glühfaden (810) zu veranlassen, elektromagnetische Strahlung auf den Zielbereich aufzubringen.

13. Lichtbasiertes System nach Anspruch 12, wobei der Photosensibilisator in einem Gel suspendiert oder aufgelöst ist oder in einer flüssigen Lösung gelöst ist.

14. Lichtbasiertes System nach Anspruch 13, wobei das Gel außerdem eine exogene Faser enthält.

15. Lichtbasiertes System nach Anspruch 13, wobei die flüssige Lösung außerdem eine exogene Faser enthält.

## Revendications

1. Procédé de réalisation d'un traitement cosmétique, le procédé comprenant :
(a) appliquer un photosensibilisateur (102) sur une fibre de kératine d'un sujet tandis que la fibre de kératine est dans un premier état physique ; et
(b) soumettre la fibre de kératine et le photosensibilisateur à un rayonnement électromagnétique (104) sélectionné pour effectuer la formation d'une liaison covalente entre deux parties différentes de la fibre de kératine individuelle pour amener la fibre de kératine à effectuer une transition vers un second état physique qui est différent du premier état physique.

2. Procédé selon la revendication 1, dans lequel la fibre de kératine est positionnée à l'intérieur d'une mèche de cheveux du sujet et lequel procédé comprenant en outre :
(a) appliquer une protéine fibreuse sur la mèche de cheveux du sujet ;
(b) soumettre la protéine fibreuse, le photosensibilisateur (102) et la mèche de cheveux à un rayonnement électromagnétique (104) choisi pour effectuer une photoréticulation entre la protéine fibreuse et la fibre de kératine à l'intérieur de la mèche de cheveux ; et
dans lequel la photoréticulation provoque une transition de la fibre de kératine du premier état physique vers le second état physique.

3. Système basé sur la lumière pour traiter et mettre en forme une fibre de kératine, le système (200) comprenant :
un système d'alimentation en fluide (204) comprenant un récipient (212) en communication fluidique avec une sortie de fluide (214) ;
un système d'activation (206) configuré avec une ou plusieurs sources de lumière (216), la source de lumière (216) étant configurée pour appliquer un rayonnement électromagnétique à une région cible qui inclut la fibre de kératine ; et
un processeur (202) ayant une mémoire (208) et configuré pour être en communication électrique avec le système d'activation (206), le processeur (202) étant configuré pour mettre en œuvre des instructions stockées dans la mémoire (208) afin d'amener le système d'activation (206) à appliquer le rayonnement électromagnétique à la région cible pour effectuer la formation d'une liaison covalente entre deux parties différentes de la fibre de kératine individuelle afin de provoquer une transition d'un premier état physique vers un second état physique,
dans lequel le récipient (212) est configuré pour contenir un photosensibilisateur et la sortie de fluide (214) est configurée pour distribuer le photosensibilisateur à la région cible.

4. Système basé sur la lumière selon la revendication 3, dans lequel le processeur (202) est en communication électrique avec un système de distribution pour amener le système de distribution à distribuer le photosensibilisateur à la région cible afin de recouvrir au moins une partie de la fibre de kératine avec le photosensibilisateur.

5. Système basé sur la lumière selon la revendication 3, comprenant en outre un dispositif cosmétique (218) configuré pour appliquer une force physique à la fibre de kératine dans la région cible afin de faire passer la fibre de kératine du premier état physique au second état physique.

6. Système basé sur la lumière selon la revendication 5, dans lequel le dispositif cosmétique (218) inclut un élément chauffant (220) ou un élément refroidissant (222).

7. Système basé sur la lumière selon la revendication 5, dans lequel le dispositif cosmétique (218) comprend :
une paire de poignées (302, 304) s'étendant à partir d'une charnière (306), la paire de poignées (302, 304) incluant une première poignée (302) et une seconde poignée (304) ;
un premier élément conducteur (308) fixé sur une surface intérieure (310) de la première poignée (302) ;
un second élément conducteur (312) fixé sur une surface intérieure (314) de la seconde poignée (304) ;
au moins un élément chauffant (220) en contact avec le premier élément conducteur (308) et le second élément conducteur (312) ;
au moins un orifice de distribution de fluide (316) configuré pour distribuer le photosensibilisateur à la région cible ;
au moins un orifice de distribution de lumière (318) configuré pour appliquer un rayonnement électromagnétique à la région cible ; et
dans lequel le processeur (202) est en communication électrique avec ledit au moins un élément chauffant (220) et est programmé pour faire augmenter la température du premier élément conducteur (308) et du second élément conducteur (312).

8. Système basé sur la lumière selon la revendication 5, dans lequel le dispositif cosmétique (218) comprend :
un corps conducteur de chaleur (402) ayant une surface extérieure et un centre creux ;
au moins un élément chauffant (220) disposé à l'intérieur du centre creux et configuré pour chauffer le corps conducteur de chaleur (402) ;
une poignée (404) couplée au corps conducteur de chaleur (402) ;
une pince (406) reliée de manière pivotante au corps conducteur de chaleur (402), la pince (406) étant mobile entre une position ouverte et une position fermée, dans lequel la pince (406) est configurée pour reposer sur une surface du corps conducteur de chaleur (402) lorsque la pince (406) est en position fermée ;
au moins un orifice de distribution de fluide (408) configuré pour délivrer le photosensibilisateur à la région cible ;
au moins un orifice de distribution de lumière (410) configuré pour appliquer un rayonnement électromagnétique à la région cible ; et
dans lequel le processeur (202) est en communication électrique avec ledit au moins un élément chauffant (220) pour amener ledit au moins un élément chauffant (220) à monter en température.

9. Système basé sur la lumière selon la revendication 5, dans lequel le dispositif cosmétique (218) comprend :
un boîtier allongé (502) s'étendant d'une entrée (504) à une sortie (506) pour définir un axe de flux d'air ;
un ventilateur positionné à l'intérieur du boîtier allongé (502) pour aspirer de l'air dans le boîtier allongé (502) par l'entrée (504) et forcer l'air à travers la sortie (506) vers une région cible ;
au moins un élément chauffant (220) disposé à l'intérieur du boîtier (502) ;
au moins un orifice de distribution de fluide (506) configuré pour distribuer le photosensibilisateur à la région cible ;
au moins un orifice de distribution de lumière (512) configuré pour appliquer un rayonnement électromagnétique à la région cible ;
le processeur (202) en communication électrique avec ledit au moins un élément chauffant (220) pour amener l'élément chauffant (220) à augmenter la température du flux d'air à travers l'axe de flux d'air.

10. Système basé sur la lumière selon la revendication 5, dans lequel le dispositif cosmétique (218) comprend :
une poignée (602) s'étendant d'une première extrémité (604) à une seconde extrémité (606) ;
au moins un filament (608) s'étendant d'une surface extérieure de la poignée (602), dans lequel la source de lumière (216) du système d'activation (206) est configurée pour amener ledit au moins un filament (608) à appliquer un rayonnement électromagnétique à la région cible.

11. Système basé sur la lumière selon la revendication 5, dans lequel le dispositif cosmétique (218) comprend :
une poignée (702) s'étendant d'une première extrémité (704) à une seconde extrémité (706) ;
un milieu poreux (708) configuré à la seconde extrémité (706) ; et
au moins un orifice de distribution de lumière (512) configuré pour appliquer un rayonnement électromagnétique à la région cible.

12. Système basé sur la lumière selon la revendication **5,** dans lequel le dispositif cosmétique (218) comprend :
un conteneur (804) configuré pour recevoir le photosensibilisateur ;
un applicateur (802) comprenant une tige (806) qui s'étend entre une tête **d'applicateur** (808) et une poignée (810), la poignée (810) étant configurée pour être couplée de manière amovible à une face supérieure du conteneur (804) et la tige (806) étant configurée pour être disposée à l'intérieur du conteneur (804) ; et
au moins un filament (810) s'étendant à partir d'une surface extérieure de la tête d'applicateur (808), dans lequel la source de lumière (216) du système d'activation (206) est configurée pour amener ledit au moins un filament (810) à appliquer un rayonnement électromagnétique à la région cible.

13. Système basé sur la lumière selon la revendication 12, dans lequel le photosensibilisateur est en suspension ou dissous dans un gel ou est dissous dans une solution liquide.

14. Système basé sur la lumière selon la revendication 13, dans lequel le gel comprend en outre une fibre exogène.

15. Système basé sur la lumière selon la revendication 13, dans lequel la solution liquide comprend en outre une fibre exogène.
